Europäisches Patentamt

European Patent Office

Office européen des brevets

(11) Publication number: 0 281 298
A2

# EUROPEAN PATENT APPLICATION

(21) Application number: 88301495.3

(22) Date of filing: 22.02.88

(51) Int. Cl.⁴: C12Q 1/18

(30) Priority: 24.02.87 US 18111
24.02.87 US 18119

(43) Date of publication of application:
07.09.88 Bulletin 88/36

(84) Designated Contracting States:
AT BE CH DE ES FR GB GR IT LI LU NL SE

(71) Applicant: MOBIL OIL CORPORATION
150 East 42nd Street
New York New York 10017(US)

(72) Inventor: Blackburn, Gary Ray
1111 General Sullivan Road
Washington Cross Pennsylvania 18977(US)
Inventor: Mackerer, Carl Robert
5 Blue Spruce Drive
Pennington New Jersey 08534(US)
Inventor: Schreiner, Ceinwen Ann
1950 Briarcliff Avenue
Meadowbrook Pennsylvania 19046(US)

(74) Representative: Colmer, Stephen Gary
Patent Department c/o Mobil Services
Company Limited Mobil Court 3 Clements
Inn
London WC2A 2EB(GB)

(54) Assaying of polynuclear aromatic compounds.

(57) A method for the assay of mutagenic activity in a hydrocarbon mixture, which comprises contacting a sample of said hydrocarbon mixture with nitric acid under conditions effective to nitrate mutagenic components thereof; recovering from said nitric acid contacted mixture a solution containing said nitrated mutagens; and determining the amount of said mutagens by determining the light absorbance of said nitrated mutagens or by incubating without added metabolic activator an inoculum of a histidine-deficient strain of Salmonella typhimurium in the presence of said recovered hydrocarbon mixture including nitrated mutagens and determining the revertant colonies so produced.

EP 0 281 298 A2

## ASSAYING OF POLYNUCLEAR AROMATIC COMPOUNDS

This invention is concerned broadly with a method for the assaying of polynuclear aromatics (PNAs). It is more specifically concerned with a rapid chemical method for the assaying of the PNAs in a complex mixture of hydrocarbons.

It is further concerned with evaluating the mutagenicity of hydrocarbon mixtures derived from petroleum. This invention, is also concerned with evaluating the carcinogenic activity of hydrocarbons. It is particularly concerned with evaluating the dermal carcinogenic activity, if any, of complex mixtures of hydrocarbons such as are encountered in a petroleum refinery, and of the fuels, lubricants and other products produced therefrom. It is more particularly concerned with a rapid method for assaying the mutagenic character of such hydrocarbon mixtures without isolation of the individual mutagenic components.

It is generally known that petroleum consists of a complex mixture of many hydrocarbons, including the so-called polynuclear aromatics. These PNAs, being relatively high boiling, are substantially absent in distillation cuts boiling below 260°C (500°F), but are found in higher boiling fractions in increasing concentration as the boiling point increases, and in the residuum. Materials classed as PNAs have from three to seven or more condensed rings, some of which may be partially saturated, and they usually have one or more alkyl and/or cycloalkyl side chains of various molecular weights. In addition, some of the PNAs contain sulfur as a heteroatom, and may only be separated from the pure hydrocarbon PNAs with great difficulty, if at all. It is also known that the content of PNAs, and probably the distribution of their molecular weights, vary with the petroleum source. See "Encyclopedia of Chemical Technology", Kirk-Othmer, 3rd Edition, Petroleum Composition, Vol. 17, pp 119-129, incorporated herein by reference, as background material.

PNAs are of importance for at least two reasons. In petroleum refining, they may adversely affect the practical properties, of certain lubricants and other products. In such instances, they usually are removed or converted to other hydrocarbon types. And, ecologically, some of the PNAs are recognized to have dermal carcinogenic activity.

The generally accepted method for evaluating the carcinogenic activity of petroleum products involves animal tests in which animals such as mice are exposed to the hydrocarbon by painting a portion of the skin repeatedly over a long period of time, and evaluating the tendency of such exposure to produce malignant growths. It is generally recognized that this test method requires seventy to eighty weeks of exposure to produce reliable results, and therefore that the method is not suited for situations in which a quick indication of potential carcinogenic activity is required.

In vitro mutagenic activity assays, such as, for example, the Salmonella Microsomal Activation Assay described by B. N. Ames, J. McCann, and E. Yamasaki in Mutat. Research, 31, 347-364 (1975), hereinafter referred to as the "Ames test", provide a rapid, inexpensive method for screening chemicals for carcinogenic potential. The entire content of this publication is incorporated herein by reference as if fully set forth. In general, the predictability of this assay with simple chemicals is good; validation studies have produced a 65-90% correlation between mutagenic activity and carcinogenic activity for many relatively pure compounds. However, the assay is unsuited to the testing of water insoluble complex mixtures, such as the complex hydrocarbon mixtures encountered in petroleum refinery streams. Attempts to use the Ames test procedure with such materials give results which are not reproducible and do not relate in a significant way to the known carcinogenic activity index for previously tested mixtures.

U.S. 4,499,187 to Blackburn et al. discloses a modification of the Ames test, hereinafter referred to as the "Modified Ames Test", suitable for use with complex hydrocarbon mixtures. The modification, in essence, involves the preparation of a DMSO (dimethylsulfoxide) extract of the sample being evaluated, and use of the DMSO extract instead of the sample itself in the Ames test together with an optimal amount of metabolic activator such as induced rat liver homogenate S-9. Detailed descriptions of the method for preparing the extract and for the assay· itself are given in U.S. 4,499,187, the entire content of which is incorporated herein by reference. The Modified Ames Test, unlike the Ames test itself, provides a rapid and reproducible measure of the mutagenic activity of petroleum hydrocarbon mixtures, and the results of such assays strongly correlate with the carcinogenic activity index found for the mixtures by skin painting.

The Modified Ames Test described above is much more rapid than skin painting, requiring only about two to three days to complete an assay compared with 18 months. There remains a need, however, for a less labor-intensive and less costly assay.

It is an object of this invention to provide a rapid, inexpensive and very sensitive chemical method for assaying the PNA content of a hydrocarbon oil. It is a further object to provide a rapid chemical method for assaying the mutagenicity and potential carcinogenic potency of a hydrocarbon oil derived from petroleum. It is a still further object of this invention to provide a rapid method for controlling certain petroleum refinery processes wherein PNAs are segregated, such as solvent extraction. These and other objects will become evident to one skilled in the art on reading this entire specification, including the appended claims.

The present invention achieves these objects by providing a method for the assay of mutagenic activity in a hydrocarbon mixture, which comprises contacting a sample of said hydrocarbon mixture with nitric acid under conditions effective to nitrate mutagenic components thereof, recovering from said nitric acid contacted mixture a solution containing said nitrated mutagens, and determining the amount of said mutagens by determining the light absorbance of said nitrated mutagens or by incubating without added metabolic activator an inoculum of a histidine-deficient strain of Salmonella typhimurium in the presence of said recovered hydrocarbon mixture including nitrated mutagens and determining the revertant colonies so produced.

It has now been found that the PNA content of a complex hydrocarbon mixture is advantageously assayed by nitrating a sample of the mixture under relatively mild conditions followed by determining the absorbance of light by the nitrated PNAs, preferably at a suitable wavelength such as 340 nm (nanometers). As will be shown hereinbelow, the absorbance of the nitrated material is proportional to the PNA content of the original oil. It has further been found that for hydrocarbon oils derived from petroleum and that boil within the range of about 260 to 538°C (500 to 1000°F), there is a very strong correlation between the absorbance of the nitrated material and both the mutagenic activity of the original oil as determined by the Ames Test and the Carcinogenic Potency determined by skin painting. Thus, the method of this invention provides a very fast and reproducible assay of the mutagenic activity of the oil.

It has also been found that the mutagenicity of a complex hydrocarbon mixture is advantageously determined by nitrating a sample of the mixture under relatively mild conditions, and subsequently assaying the mutagenicity in the Ames Test without use of a metabolic activator, all as more fully described hereinbelow. Because the nitration step eliminates the need for a liver metabolizing system, and performance of the assay is less labor-intensive, cost reduction is achieved without sacrifice of reliability and without the use of animal tissue. As will be shown hereinbelow, the method is particularly well suited to the assay of complex mixtures of hydrocarbons derived from petroleum and provides reliable predictability of the presence or absence of dermal carcinogenic activity for a mixture that boils above 260°C.

Although it is contemplated that the method of this invention may be used to reliably determine the mutagenicity of a complex mixture of hydrocarbons from any source, it is particularly useful for rapidly estimating the potential dermal carcinogenicity of a complex hydrocarbon mixture derived from petroleum. As will be shown below, there is a very strong correlation between the absorbance at 340 nm derived by the method of this invention and carcinogenic potency for petroleum derived mixtures. The method of this invention is useful for petroleum derived mixtures substantially free of material boiling below 260°C, i.e. containing less than about 10 vol. % of material boiling below 260°C. For those that have a significant component boiling below 260°C, the correlation has been observed to deteriorate. With such mixtures, it is preferred to isolate the fraction boiling above 260°C prior to nitration. The method of this invention is particularly useful for petroleum derived mixtures boiling within the range of about 260°C to about 538°C. For those oils that have a significant component boiling below 260°C and/or above 538°C, the correlation has been observed to deteriorate. With such mixtures, it is preferred to isolate the fraction boiling from about 260°C to about 538°C prior to nitration. All references made herein to boiling point are to be understood to refer to the boiling point as determined by ASTM Method D-1160 (Distillation of Petroleum Products at Reduced Temperatures), published by the American Society for Testing Materials, 1916 Race Street, Philadelphia, Pennsylvania.

Although DMSO is the presently preferred solvent for the nitrated material, it is contemplated that other solvents such as those approved for use in the Ames Assay and/or those effective in solubilizing the nitrated oil also may be used.

The term "derived from petroleum" as used herein is intended to include petroleum fractions obtained by physical methods such as distillation, solvent extraction, and the like as well as such fractions that have been subjected to petroleum processes such as clay treating, hydrocracking, hydrofinishing, and the like.

The term "absorbance" is used herein in the conventional sense and refers to log (I₀/I) wherein I₀ and I are the intensities of the incident light and the transmitted light, respectively. All measurements reported herein are based on the same cell thickness.

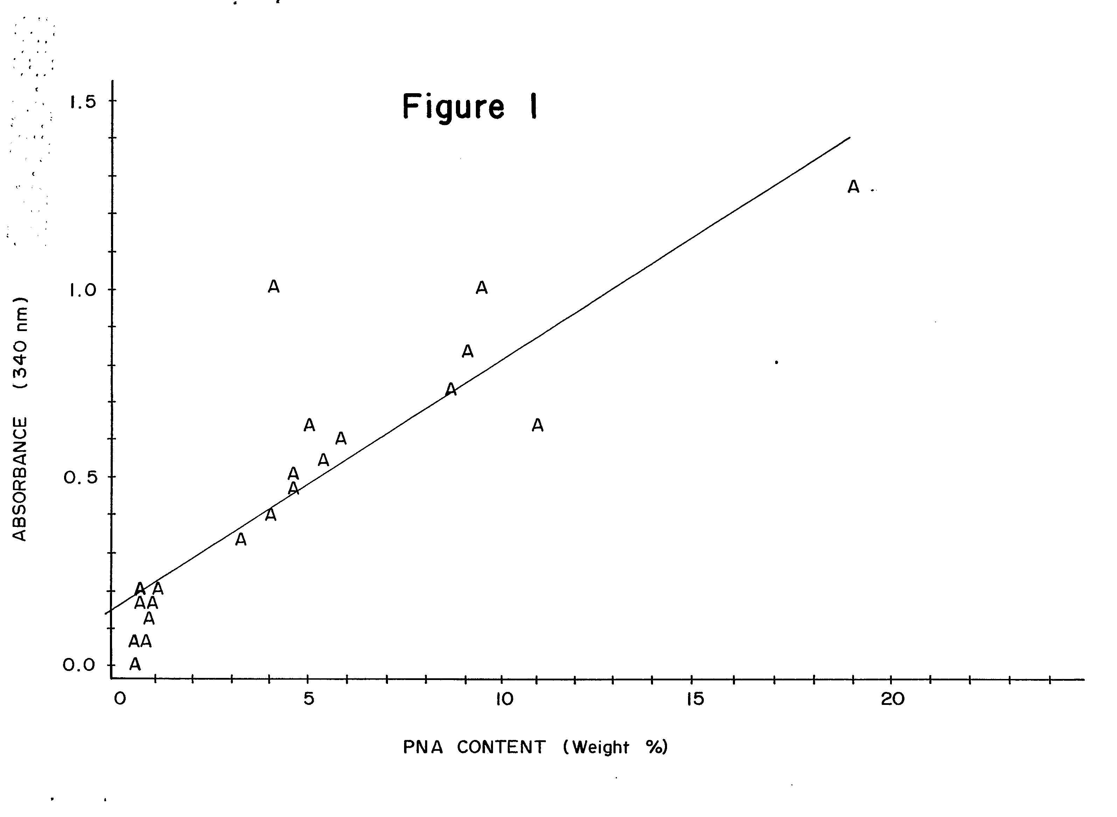

In the drawings, FIG. 1. is a correlation of Absorbance at 340 nm with gravimetrically determined PNAs;

FIG. 2. is a correlation of Absorbance at 340 nm with mutagenicity by Ames Test;

FIG. 3. is a correlation of Absorbance at 340 nm with Carcinogenic Potency from skin painting;

FIG. 4. is a correlation of Carcinogenic Potency with Mutagenicity Index determined by the Modified Ames Test (Prior Art);

FIG. 5. is a correlation of Mutagenicity Indices determined by the method of the present invention and by Modified Ames Test; and

FIG. 6. is a correlation of Carcinogenic Potency with Mutagenicity Index determined by method of the present invention.

In the drawings the points labelled "A" are observations measured once and the points labelled "B" are observations measured twice.

This invention will now be illustrated by Examples. The Examples, however, are not to be construed as limiting the scope of the invention, which scope is determined by this entire specification including the appended claims.

EXAMPLE 1

A sample of the oil to be assayed is nitrated by the following procedure. To a known weight or volume of oil in about the 10 milligram range, and contained in 0.1 ml of cyclohexane, is added 1 ml of 80% nitric acid of reagent grade or better. This mixture is incubated at 80°C for two hours. It is then neutralized by adding 1 ml of 10 Molar sodium hydroxide. The neutralized mixture is cooled and 5 ml of dichloromethane is added, mixed, and allowed to stand for phase separation. The upper, aqueous phase is then carefully withdrawn and discarded, particular attention being given to avoid losses of any of the dichloromethane phase. 100 microliters of the dichloromethane extract is withdrawn and added to 0.9 ml of DMSO. The absorbance of the DMSO solution, referred to hereinbelow as the "test sample", is then determined with a spectrophotometer at 340 nm.

EXAMPLE 2

A sample of the oil to be assayed is nitrated by the following procedure. To a known weight or volume of oil in about the 10 mg range, an contained in 0.1 ml of cyclohexane, is added 1 ml of 80% nitric acid of reagent grade or better. This mixture is incubated at 80°C for two hours. It is then neutralized by adding 1 ml of 10 Molar sodium hydroxide. The neutralized mixture is cooled and 5 ml of dichloromethane is added, mixed, and allowed to stand for phase separation. The upper, aqueous phase is then carefully withdrawn and discarded, particular attention being given to avoid losses of any of the dichloromethane phase. After the initial separation, the dichloromethane phase is washed with 1 ml of water to remove some of the salts that are present in the residual aqueous phase, and the wash liquid removed and discarded. The dichloromethane phase is then evaporated and the recovered residue, which usually has a yellow to brown-red color, is dissolved in 2 ml of DMSO. This solution, referred to hereinbelow as the "test sample", is then asayed in the Ames Test, without addition of a liver metabolic activation system, and preferably with the use of the histidine-deficient mutant strain of Salmonella typhimurium TA98.

The Ames Test is conducted by introducing into a series of sterile capped culture tubes up to about eight different doses of the test sample. Whenever necessary, the test sample is diluted as required to insure accurate deiivery The doses are selected to delimit the linear portion of the dose-response curve. To facilitate dosage selections, it is usually desirable to conduct a preliminary assay to approximately establish the boundaries of the dosages for the linear response range. To each tube the following are added: the appropriate volume of the test sample in DMSO; 0.5 ml of 0.2 Molar sodium phosphate buffer, pH 7.4; and 0.1 ml of Salmonella typhimurium TA98 broth culture ($1 \times 10^9$ cells per ml). Duplicate or triplicate tubes are prepared for each dose level. The cultures are incubated at 37°C with agitation for 20 minutes, following which top agar (0.6% agar, 0.5% NaCl, 0.5 mM histidine-biotin) is added. The tubes are vortexed to ensure adequate mixing and the contents overlaid on 30 ml Vogel-Bonner minimal media plates. Plates are incubated inverted at 37°C in the dark and revertant colonies are counted 48 hours later.

Mutagenic activity is assessed from the slope of the linear portion of the dose response curve that is obtained, and is reported as revertants per microliter of test sample.

The nitration procedure described above is very effective for purposes of the present invention, and is that which was used to establish the correlations generated by the Examples described below, unless otherwise noted. The Examples illustrate the preferred method of nitration. However, modifications of the nitration method may be used. For example, a sample may be nitrated at room temperature for 10 minutes with concentrated nitric acid (approximately 16 Molar) after which the nitrated mixture is neutralized and further treated as above to provide the test sample.

Although the histidine-deficient <u>Salmonella typhimurium</u> tester strain TA98 originally developed by B. N. Ames at the University of California, Berkley, is particularly preferred, it is contemplated that other histidine-deficient strains of the organism such as TA100 may be useful.


## EXAMPLE 3

A data base was established using a series of twenty-one different petroleum oils and by-product extracts. These all boiled above about 260°C, and all but Sample No. 5 boiled within the range of about 260°C to 538°C. Sample No. 5, the hydrotreated bright stock extract, contains components which boiled above 538°C. Table I provides a description for each of the twenty-one oils.

Each of the oil samples was evaluated for mutagenicity by the Modified Ames Test using the slope of the linear portion of the dose-response curve, as described above, and by the method of this invention, either by measuring the absorbance at 340 nm or by calculating the Mutagenicity Index determined from the slope of the linear portion of the dose-response curve, as described above. Table II summarizes the results of the Mutagenicity Tests, and also the results of skin painting tests in terms of the observed LP (latent period), i.e. the average number of weeks elapsed before the development of a tumor, the number of animals that developed tumors, and the Carcinogenic Potency calculated as 100/LP. Oils that produced no tumors in 100 weeks are arbitrarily assigned a Carcinogenic Potency = 1.00.

Table II also shows the gravimetrically determined total PNA concentration as wt% of each oil. These values were obtained gravimetrically from the DMSO extracts of the oils, using a method similar to the one described for this purpose by T.A. Roy et al, Estimation of Mutagenic and Dermal Carcinogenic Activities of Petroleum Fractions based on Polynuclear Aromatic Hydrocarbon Content, Polynuclear Aromatic Hydrocarbon: A Decade of Progress, Edited by Marcus Cooke and Anthony J. Dennis, Battelle Press, Columbus, Ohio, pages 809-824 (1988).

It is here noted that not every one of the 21 samples described in Table I were included in Examples 4-6 which follow.

Sample

| No. | Description |
|---|---|
| 1 | Hydrotreated Machine Oil Extract |
| 2 | Hydrotreated Machine Oil Extract |
| 3 | Hydrotreated Machine Oil Extract |
| 4 | Hydrotreated Machine Oil Extract |
| 5 | Hydrotreated Bright Stock Extract |
| 6 | Furfural Extracted Naphthenic Distillate |
| 7 | Technical White Oil (SO$_2$ Extracted/Hydrofinished) |
| 8 | Furfural Extracted/Ferrofined Paraffinic Distillate |
| 9 | Mildly Furfural Extracted/Polished Paraffinic Distillate |
| 10 | Light Medicinal Oil BP |
| 11 | Furfural Extracted/Polished Paraffinic Distillate |
| 12 | Distillate Aromatic Extract |
| 13 | SO$_2$/Benzene Extracted, Ferrofined Paraffinic Distillate |
| 14 | Acid/Earth Treated Naphthenic Distillate |
| 15 | SO$_2$ Extracted Naphthenic Distillate |
| 16 | SO$_2$ Extracted, Earth Finished Naphthenic Distillate |
| 17 | Mildly Hydrotreated Naphthenic Distillate |
| 18 | Hydrotreated Neutralized Naphthenic Distillate |
| 19 | Hydrotreated Naphthenic Distillate |
| 20 | Low Viscosity Index Paraffinic Oil - Hydrotreated |
| 21 | Low Viscosity Index Paraffinic Oil - Hydrotreated |

| A | B | C | D | E | F | G | H |
|---|---|---|---|---|---|---|---|
| 1 | 9.7 | 140 | 1.02 | 37 | 22 | 2.70 | 9.3 |
| 2 | 11.0 | 106 | .831 | 36 | 23 | 2.78 | 9.0 |
| 3 | 5.2 | 95 | 1.02 | 55 | 7 | 1.82 | 4.0 |
| 4 | 4.6 | -- | .649 | 70 | 6 | 1.43 | 5.0 |
| 5 | 0.0 | -- | .475 | -- | 0 | 1.00 | 4.5 |
| 6 | 0.0 | 0 | .216 | -- | 0 | 1.00 | 0.7 |
| 7 | 0.0 | 0 | .058 | -- | 0 | 1.00 | 0.5 |
| 8 | 0.9 | 7 | .198 | 48 | 1 | 2.08 | 0.9 |
| 9 | 0.0 | -- | .165 | -- | 0 | 1.00 | 0.6 |
| 10 | 0.0 | 0 | .007 | -- | 0 | 1.00 | 0.3 |
| 11 | 0.0 | 10 | .139 | -- | 0 | 1.00 | 0.7 |
| 12 | 17.0 | 354 | 1.26 | 26 | 41 | 3.85 | 19.0 |
| 13 | 2.4 | 70 | .340 | 44 | 2 | 2.27 | 3.2 |
| 14 | 9.1 | 284 | .644 | 20 | 6 | 5.00 | 11.0 |
| 15 | 0.0 | 0 | .176 | 58 | 1 | 1.72 | 0.7 |
| 16 | 0.0 | 0 | .072 | -- | 0 | 1.00 | 0.4 |
| 17 | 3.9 | 114 | .485 | 46 | 1 | 2.17 | 4.6 |
| 18 | 4.0 | 95 | .393 | 56 | 4 | 1.79 | 4.0 |
| 19 | 3.6 | 123 | .535 | 46 | 5 | 2.17 | 5.4 |
| 20 | 6.5 | 178 | .589 | 43 | 5 | 2.33 | 5.8 |
| 21 | 9.2 | 170 | .728 | 29 | 20 | 3.45 | 8.5 |

A = Sample Number

B = Mutagenicity Index (Modified Ames)

C = Mutagenicity Index (Nitrated Material)

D = Absorbance (340 nm)

E = Latent Period (LP) (weeks)

F = Number of Animals with tumor(s)

G = Carcinogenic Potency (100/LP)

H = Weight Percent PNA's (Gravimetric)

## EXAMPLE 4

In this example the correlation of the absorbance at 340 nm and the gravimetrically determined total PNAs is shown in Figure 1. The calculated sample correlation coefficient for the relationship is 0.87. In this and subsequent examples, the least squares regression line and the sample correlation coefficient are computed by the conventional procedures as given, e.g., in Statistics Manual, Edwin L. Crow et al., pp 152-159, Dover Publications, New York, N.Y. (1956), incorporated herein by reference.

## EXAMPLE 5

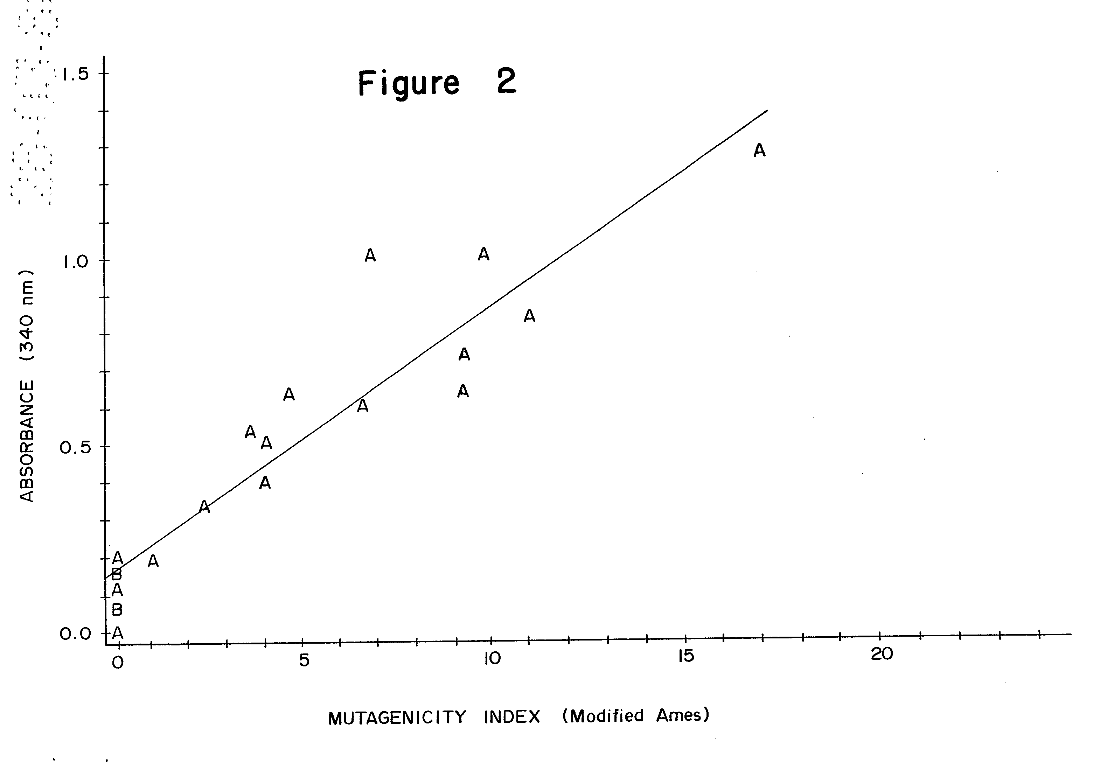

The correlation of the absorbance at 340 nm obtained by the method of this invention and shown in Table II with the Mutagenicity Index obtained by the Ames Test is shown in Figure 2. The calculated sample correlation coefficient is 0.93.

## EXAMPLE 6

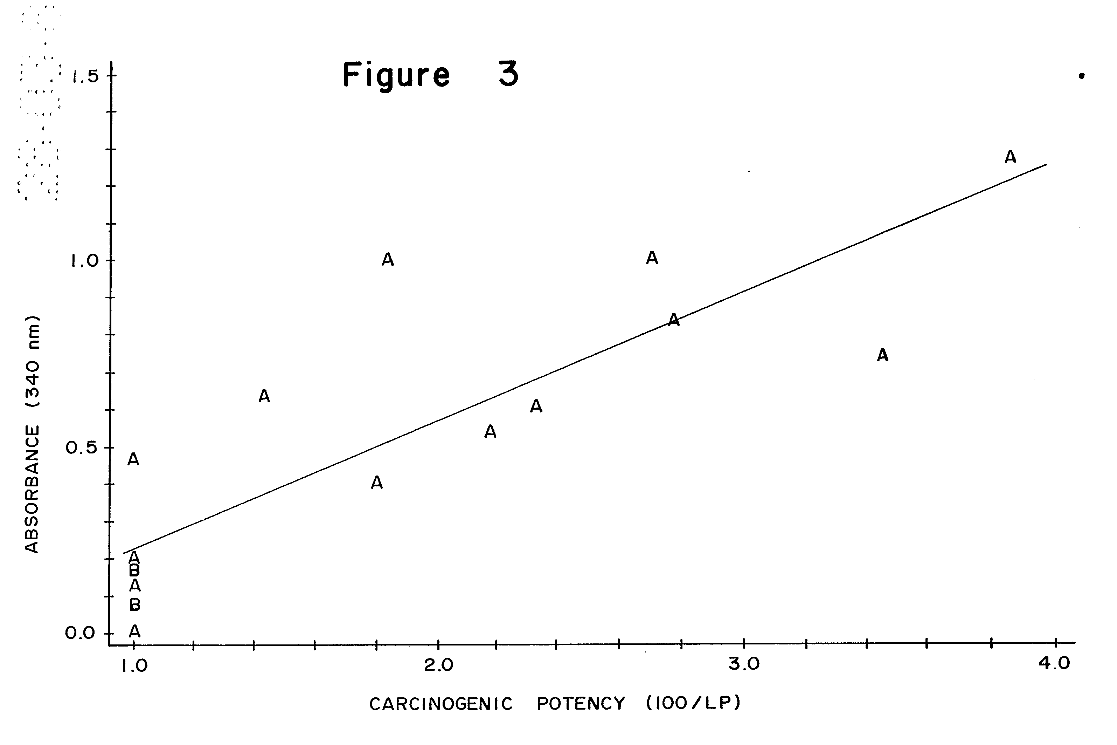

The correlation of the absorbance at 340 nm obtained by the method of this invention and the Carcinogenic Potency by skin painting are shown in Figure 3. The calculated sample correlation coefficient is 0.84.

## EXAMPLE 7 (Prior Art)

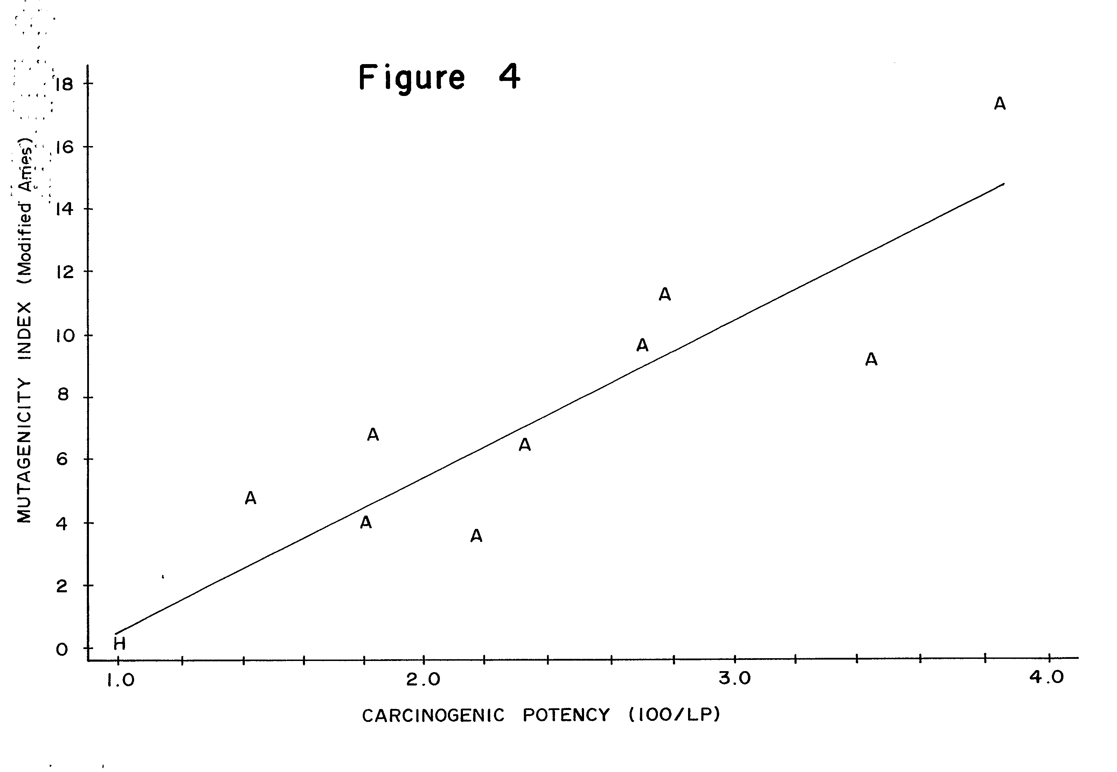

In this example the correlation of the Mutagenicity Index determined by the Modified Ames Test and the Carcinogenic Potency determined by skin painting are shown in Figure 4. The calculated sample correlation coefficient for the relationship is 0.95. In this and subsequent examples, the least squares regression line and the sample correlation coefficient are computed by the conventional procedures as given, e.g., in Statistics Manual, Edwin L. Crow et al., pp 152-159, Dover Publications, New York, N.Y. (1956), incorporated herein by reference.

## EXAMPLE 8

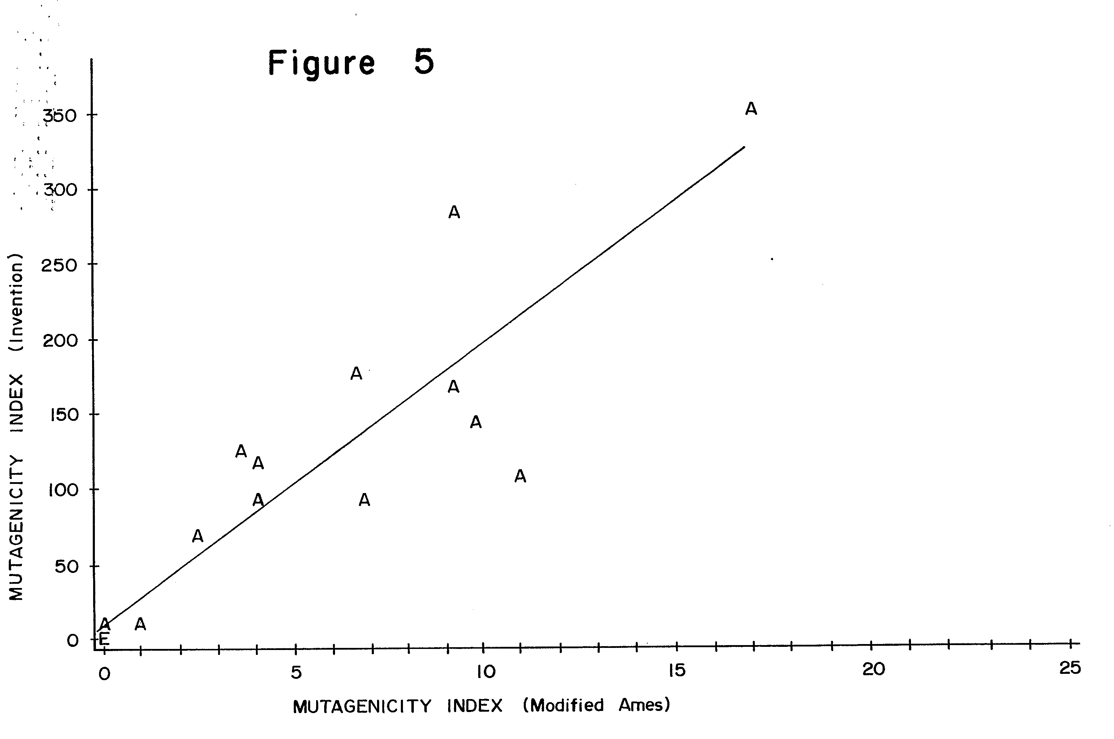

The correlation of the Mutagenicity Index obtained by the method of this invention and shown in Table II with the Mutagenicity Index obtained with the Modified Ames Test is shown in Figure 5. The calculated sample correlation coefficient is 0.90.

## EXAMPLE 9

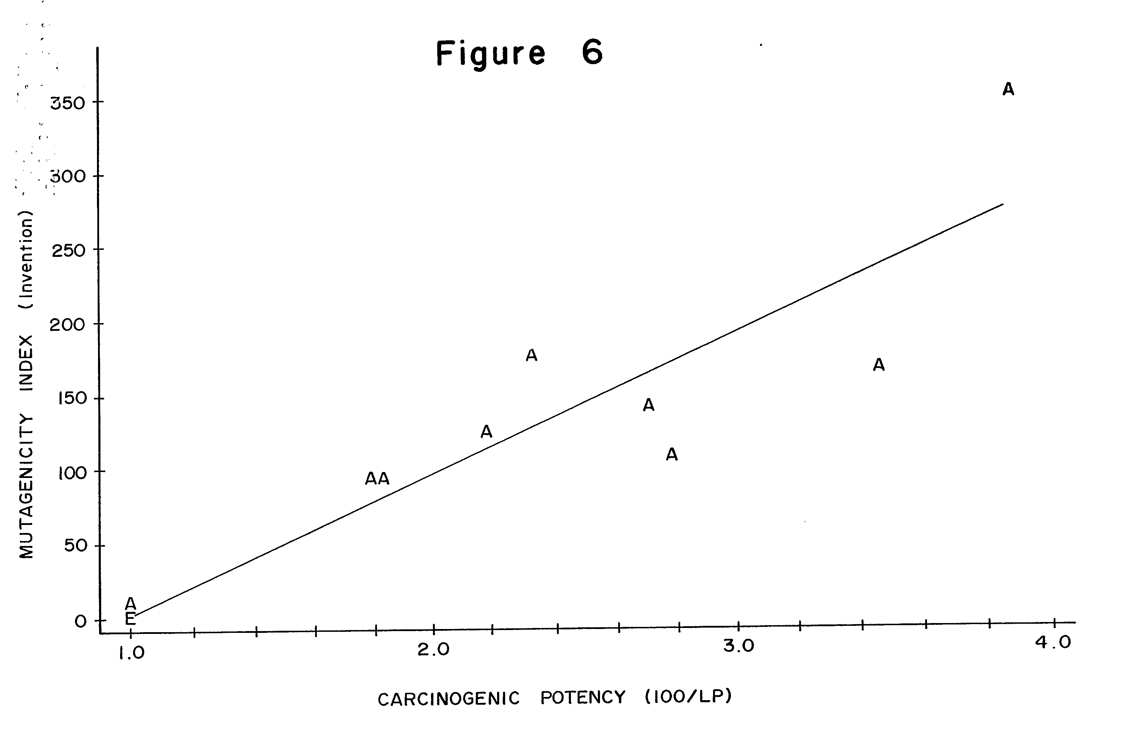

The correlation of the Mutagenicity Index obtained by the method of this invention and the Carcinogenic Potency by skin painting are shown in Figure 6. The calculated sample correlation coefficient is 0.93.

It is evident from the foregoing Examples that the method of this invention provides a sensitive assay of total PNAs, as well as a test for mutagenicity which is rapid, simpler, and much less costly than the Ames Test, and yet it provides highly significant prediction of carcinogenic potency. While not wishing to be bound by theory. it is believed that the observed correlations result from the mutagenic polynuclear aromatics contained in the oils evaluated.

It is evident from the foregoing Examples that the method of this invention provides a test for mutagenicity which is simpler and more sensitive than the Modified Ames Test and yet provides highly significant prediction of carcinogenic potency. While not wishing to be bound by theory, it is believed that the observed correlations result from the mutagenic polynuclear aromatics contained in the oils evaluated.

Although the method of this invention has been exemplified with hydrocarbon mixtures derived from petroleum, it is contemplated that the method may be used with hydrocarbon mixtures derived from other fossil fuels such as coal and tar sands.

Because determinations of PNA content by the method of this invention are very sensitive and very rapid, requiring about 20 minutes for an assay, the method lends itself not only to the determination of mutagenicity as described above, but also to other applications for which a determination of the PNA content may be relevant. For example, in the solvent extraction process as applied to heavy oils to selectively remove aromatics, the quality of the product and the efficiency of the process may be advantageously monitored by the method provided herein. It is also contemplated to monitor environmental samples for relative PNA content and potential mutagenicity by use of the method of this invention.

## Claims

1. A method for the assay of mutagenic activity in a hydrocarbon mixture, which comprises:

contacting a sample of said hydrocarbon mixture with nitric acid under conditions effective to nitrate mutagenic components thereof;

recovering from said nitric acid contacted mixture a solution containing said nitrated mutagens; and

determining the amount of said mutagens by determining the light absorbance of said nitrated mutagens or by incubating without added metabolic activator an inoculum of a histidine-deficient strain of Salmonella typhimurium in the presence of said recovered hydrocarbon mixture including nitrated mutagens and determining the revertant colonies so produced.

2. A rapid method for the assay of the polynuclear aromatics in a hydrocarbon mixture, which method comprises:

contacting a sample of said hydrocarbon mixture with nitric acid under conditions effective to nitrate said polynuclear aromatics;

recovering from said nitric acid contacted mixture a solution containing said nitrated polynuclear aromatics; and

determining the light absorbance of said recovered nitrated polynuclear aromatics.

3. The method of Claim 2, wherein said hydrocarbon mixture is petroleum derived.

4. The method of Claim 3, wherein said hydrocarbon mixture is substantially free of material boiling below 260°C and of material boiling above 538°C.

5. The method of Claim 2, wherein said determination of light absorbance is made with a spectrophotometer at 340 nm.

6. The method of Claim 2, wherein said sample of said hydrocarbon mixture contains a hydrocarbon mixture dissolved in an organic solvent.

7. The method of Claim 2, wherein said organic solvent is cyclohexane.

8. The method of Claim 2, which further comprises neutralizing said nitric acid contacted mixture with an aqueous basic solution, adding an organic solvent in which said polynuclear aromatics are not soluble and which is not miscible with water to said neutralized mixture, allowing said organic solvent containing said nitrated polynuclear aromatics to separate from an aqueous phase and determining the light absorbance of nitrated polynuclear aromatics in said organic solvent.

9. A method for evaluating the mutagenic activity of a hydrocarbon mixture, which method comprises:

contacting a sample of said hydrocarbon mixture with nitric acid under conditions effective to nitrate the mutagenic components thereof;

recovering from said nitric acid contacted mixture a hydrocarbon mixture including nitrated mutagens;

determining the light absorbance of said recovered nitrated mutagens; and

comparing said absorbance with the absorbance of similarly formed nitrated material from an oil of known mutagenic activity.

10. A method for evaluating the mutagenic activity of a hydrocarbon mixture, which method comprises:

contacting a sample of said hydrocarbon mixture with nitric acid under conditions effective to nitrate mutagenic components thereof;

recovering from said nitric acid contacted mixture a hydrocarbon mixture including nitrated mutagens;

incubating without added metabolic activator an inoculum of a histidine-deficient strain of Salmonella typhimurium in the presence of said recovered hydrocarbon mixture including nitrated mutagens; and

determining the revertant colonies so produced.

11. The method described in Claim 10, wherein several incubations are conducted with different amounts of said recovered hydrocarbon mixture, and wherein the determination of revertant colonies is made from the linear portion of the dose response curve.

12. The method described in Claim 10, wherein said mutant strain of Salmonella typhimurium is TA98.

13. The method described in Claim 10, wherein said hydrocarbon mixture is petroleum derived.

14. The method described in Claim 10, wherein said hydrocarbon mixture is petroleum derived and is substantially free of material boiling below 260°C.

15. A method for determining the carcinogenic potency of a hydrocarbon mixture derived from petroleum, which method comprises:

contacting a sample of said hydrocarbon mixture with nitric acid under conditions effective to nitrate the mutagenic components thereof;

recovering from said nitric acid contacted mixture a hydrocarbon mixture including nitrated mutagens;

incubating without added metabolic activator an inoculum of a histidine-deficient strain of <u>Salmonella typhimurium</u> in the presence of said recovered hydrocarbon mixture including nitrated mutagens; and

determining the revertant colonies so produced.

16. The method described in Claim 15, wherein said hydrocarbon mixture is substantially free of material boiling below 260°C, and wherein said mutant strain of <u>Salmonella typhimurium</u> is TA98.

# Figure I

ABSORBANCE (340 nm)

PNA CONTENT (Weight %)

Figure 2

ABSORBANCE (340 nm)

MUTAGENICITY INDEX (Modified Ames)

0 281 298

# Figure 3

Figure 3

# Figure 4

Figure 5

MUTAGENICITY INDEX (Invention) vs MUTAGENICITY INDEX (Modified Ames)

# Figure 6